# EUROPEAN PATENT SPECIFICATION

(11) **EP 4 528 347 B1**
(45) Date of publication and mention of the grant of the patent: **20.05.2026**
(21) Application number: 24201352.2
(22) Date of filing: 19.09.2024
(51) Int. Cl.: G02B 7/00, G02B 21/00

(54) **DAMPER AND SURGICAL IMAGING DEVICE**
DÄMPFER UND CHIRURGISCHE BILDGEBUNGSVORRICHTUNG
AMORTISSEUR ET DISPOSITIF D'IMAGERIE CHIRURGICALE

(30) Priority: 19.09.2023 DE 102023125411
(43) Date of publication of application: 26.03.2025
(73) Proprietor: Leica Instruments (Singapore) Pte. Ltd., Singapore 618299 (SG)
(72) Inventor: FRICK, Roman, 618299 Singapore (SG)
(74) Representative: 2SPL Patentanwälte PartG mbB

(56) References cited:
- WO-A1-98/53244
- JP-B2- 6 159 706
- US-A1- 2002 020 788
- US-A1- 2002 166 942
- US-A1- 2004 262 866
- US-B2- 10 251 717

## Description

### Technical Field

The present disclosure relates to a damper for a surgical imaging device and a surgical imaging device.

### Background

In an operating room, surgical imaging devices, such as surgical microscopes, are subjected to various impacts, especially in their use time during surgery. Aside from chemical stress caused by fluid or chemical spills, the surgical imaging device comprises components, such as a fan for cooling of electronic parts or a ventilation system for drape suction, which cause slight vibration of the surgical imaging device. Usually, these vibrations can be overcome by image stabilization.

However, significant mechanical stress may also be exerted by the user while handling the surgical imaging device with or without his intend. For example, the user might accidentally hit parts of the surgical imaging device when moving around during a surgical procedure. These impacts may result in considerable or even heavy shaking of the surgical imaging device. Thereby, the user may be precluded from obtaining optimal imaging data from the operating site until the surgical imaging device returns to a steady state. The user might even have to wait until the surgical imaging device returns to a still before he might use it again for the intended surgical procedure.

US 2002/166942 A1 addresses torsional vibrations in surgical microscope stands. To address the torsional vibrations, torsional damping elements are positioned at critical rotational joints, particularly where the horizontal carrier arm connects to the vertical column. In some configurations, the damping element is installed between flanges in the brake assembly, allowing slight movement while absorbing torsional vibrations.

WO 9853244 A1 discusses a microscope stand for a surgical microscope. The invention features at least one support made from a composite material, which is constructed using two tubular materials with differing resistance properties. The support includes vibration damping mechanisms between adjacent supports or their elements.

Therefore, further efforts are required for dealing with mechanical stress exerted on the surgical imaging device.

### Summary

There may be a desire for an improved concept for damping mechanical impacts, which are exerted on the surgical imaging device.

This desire is addressed by the subject-matter of the independent claims.

Embodiments of the present disclosure provide a damper for a surgical imaging device. The damper comprises a stack of layers comprising: a first outer layer, a central layer, and a second outer layer. The layers are linked to form a base piece of the damper. The first outer layer further forms a first end of the damper configured to be connected to a stand of the surgical imaging device and the second outer layer further forms a second end of the damper configured to be connected to a base of the surgical imaging device. The damper is configured to undergo elastic deformation in response to a pushing or pulling force exhibited on one of the ends such that the central layer, but not the outer layers, undergo elastic deformation. Application of the force to the first end causes a deferral of the first outer layer relative to the second outer layer, thereby inducing shearing deformation of the central layer. The ends are extending from the same side of the base piece of the damper. The second end is configured to be movably connected to the first end. The first end is configured to be fixed at the stand by a fixation element. The second end is configured to be fixed at the base by at least one further fixation element. The second end is further configured to be mounted on an axis, with the axis being configured to engage with the fixation element in such way that during deformation the first end is displaced along the axis thereby deferring the first outer layer in direction of the force

With the damper being configured to be mounted inside the surgical imaging device such that the base and the stand are connected to one of the ends of the damper, a movement of the stand and/or the base is acting on the damper resulting in its shearing deformation. The movement may be caused by a mechanical impact on the surgical imaging device. This may occur for example when a user accidentally hits parts of the surgical imaging device during a medical procedure. Such parts may be an arm or an image acquisition apparatus carrier, like an optics carrier or a camera, of the surgical imaging device. These impacts may then be transmitted through the surgical imaging device as the respective parts, the stand and the base are mechanically linked. By that a movement of the stand and/or the base may be generated, which creates the pushing or pulling force that is acting on at least one of the ends of the damper. As a result, the damper undergoes shearing deformation, thereby changing the shape of the central layer, but not of the outer layers. It is understood that shearing deformation encompasses shearing and deshearing of the damper. In this context, deshearing is a spontaneous return of the damper to its undeformed state after its deformation or shearing. By returning to its undeformed state, not only the deformation of the damper is reversed, but also the movement of the stand and/or base, which are connected to the ends of the damper. Hence, the damper contributes to a return of the surgical imaging device to a steady state following an impact. This improves usability of the surgical imaging device during a medical procedure as the user may be able to operate the surgical imaging device instantly or at least quickly after an impact has occurred.

Embodiments of the present disclosure further provide a surgical imaging device. The surgical imaging device comprises a stand, a base, and the damper. The surgical imaging device further comprises the fixation element for fixing the first end of the damper to a housing of the stand and the further fixation element for fixing the second end of the damper to a connector part of the base. The connector part is configured to connect the base with the stand. The surgical imaging device further comprises the axis configured to movably connect the first end and the second end.

The surgical imaging device may be a surgical microscope placed in an operating room. The surgical imaging device may further comprise an arm and an image acquisition apparatus carrier, like an optics carrier or a camera. During a surgical procedure medical personnel, such as surgeons or surgery assistants, move around the surgical imaging device and, by chance, may hit one of the aforementioned parts of the surgical imaging device causing a mechanical impact. By that a pushing or pulling for is exerted on the respective part. The force is then transmitted through the surgical imaging device. Eventually, the pushing or pulling force is transmitted to the damper, which is connected to the stand and the base by its ends. Through shearing of the damper in response to the force and subsequent spontaneous deshearing, the damper contributes to a return of the surgical imaging device to a steady state after such impact has occurred.

### Short Description of the Figures

Some examples of apparatuses and/or methods will be described in the following by way of example only, and with reference to the accompanying figures, in which
- Fig. 1: shows a schematic perspective view of a damper according to an embodiment;
- Fig. 2: shows the damper of Fig. 1 in a schematic side view;
- Fig. 3: shows a schematic perspective view of the damper of the preceding figures in a deformed state;
- Fig. 4: shows the damper of Fig. 3 in a schematic side view;
- Fig. 5: shows a schematic side view of a surgical imaging device according to an embodiment;
- Fig. 6: shows a schematic perspective view of the surgical imaging device of Fig. 5 with the damper of the preceding figures;
- Fig. 7: shows another schematic perspective view of the damper of the preceding figures mounted within the surgical imaging device of Fig. 5;
- Fig. 8: shows the damper inside the surgical imaging device as in Fig. 6 and 7 in a schematic sectional view;
- Fig. 9: shows another schematic sectional view of the damper inside the surgical imaging device as in Fig. 6 and 7;
- Fig. 10: shows a schematic sectional view of the surgical imaging device with the damper of the preceding figures.

### Detailed Description

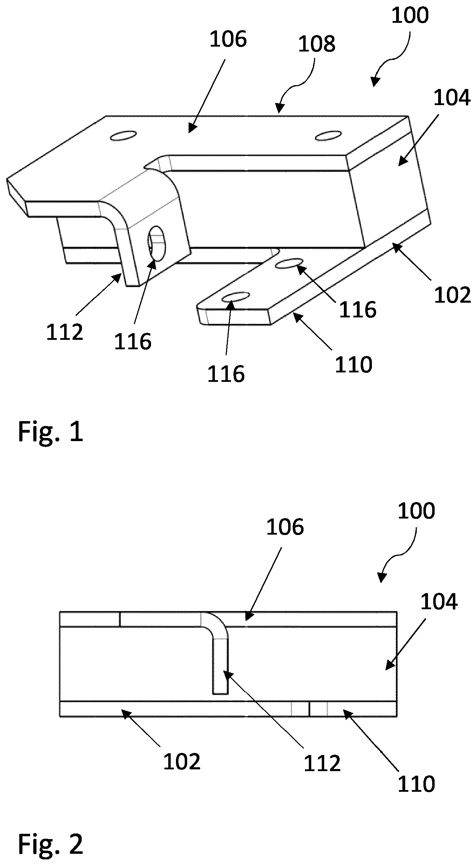

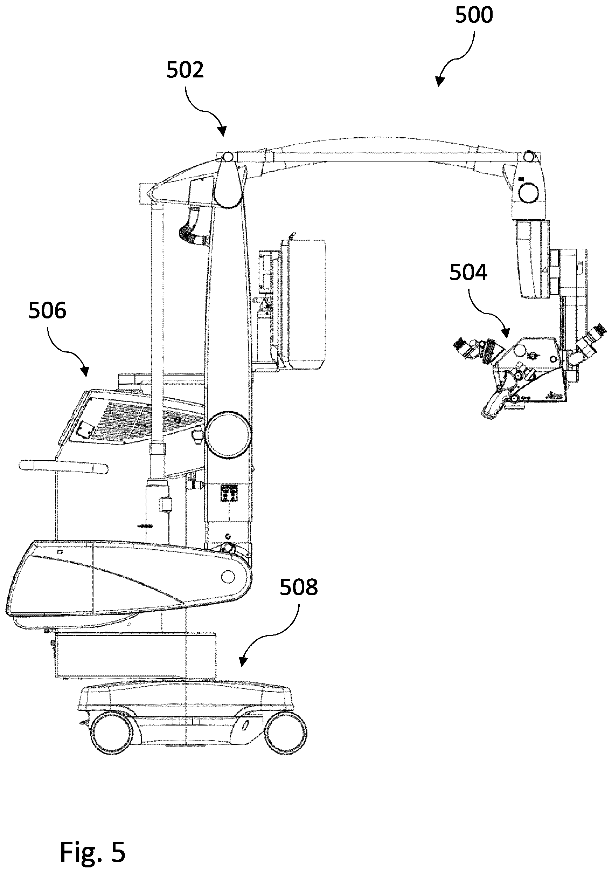

A surgical imaging device 500 (as shown in **Fig. 5**), such as a surgical microscope, may be used in an operating room during a medical procedure. A user of the surgical imaging device 500, like a surgeon or surgery assistant, may thereby accidentally hit parts of the surgical imaging device 500. These parts of the surgical imaging device 500 may comprise an arm 502 and an image acquisition apparatus carrier 504, like an optics carrier or a camera, a stand 506, and a base 508. Due to the mechanical linking, hitting of one of the aforementioned parts results in a transmission of the applied force through the surgical imaging device 500. To suppress a shaking or movement of the surgical imaging device 500 resulting from the transmitted force, the surgical imaging device 500 may further comprise a damper 100 as further described in the embodiments in **Fig. 1ff.**

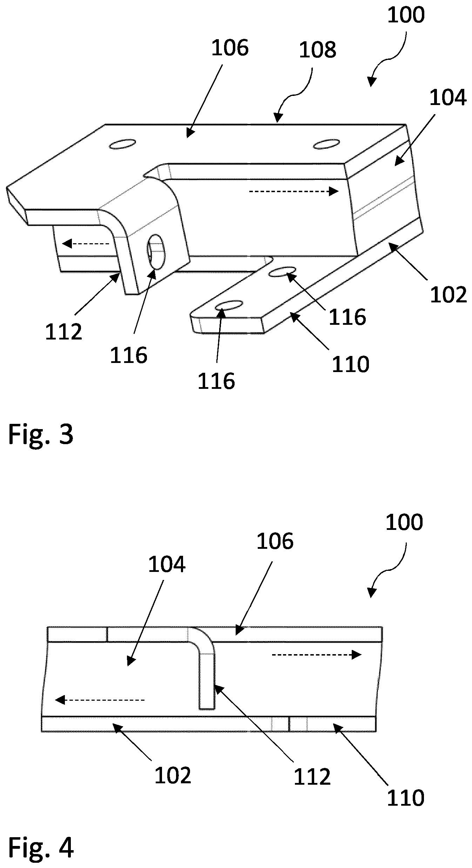

**Fig. 1** to **Fig. 4** show perspective views of a damper 100 according to an embodiment. **Fig. 1** and **Fig 2** show the damper 100 in an undeformed (resting or initial) state and **Fig. 3** and **Fig 4** show the damper 100 a deformed state.

The damper 100 comprises a stack of layers 102, 104, 106. The stack comprises a first outer layer 102, a central layer 104, and a second outer layer 106. The layers 102, 104, 106 are linked to form a base piece 108 of the damper 100. Linking of the layers 102, 104, 106 can be realized by a material or form fitting connection, which is known by a person skilled in the art. The first outer layer 102 further forms a first end 110 of the damper 100 configured to be connected to the stand 506 of a surgical imaging device 500 (as shown in Fig. 5ff). The second outer layer 106 further forms a second end 112 of the damper 100 configured to be connected to the base 508 of the surgical imaging device 500 (as shown in Fig. 5ff). By this connection, a pushing or pulling force exerted on the stand 506 and/or the base 508 acts on the damper 100 as it is transmitted via the ends 110, 112. The damper 100 is configured to undergo elastic deformation in response to that pushing or pulling force such that the central layer 104, but not the outer layers 102, 106, undergo elastic deformation.

In a resting state (as shown in **Fig. 1** and **Fig. 2**) the damper 100 and its central layer 104 remain undeformed as no or no substantial force is applied on the ends 110, 112. However, in case of a mechanical impact on the surgical imaging device 500, e.g., from a user hitting parts of the surgical imaging device 500, a movement of the stand 506 and/or the base 508 may occur. Due to the connection between damper 100 and the stand 506 and the base 508 a pushing or pulling force at the ends 110, 112 is created. An application of such force to the first end 110 results in a deferral of the first outer layer 102 relative to the second outer layer 106, thereby inducing shearing deformation of the central layer 104 (as shown in **Fig. 3** and **Fig. 4****,** arrows with dashed line indicate the deformation). To remain undeformed, the outer layers 102, 106 may be made of sheet metal. The central layer 104 may be made of an elastomer, preferably polyurethane, to be deformable. The deformation may occur in the direction of the force, so that the central layer 104 may undergo shearing deformation coaxially to a displacement of the first end 110. In further embodiments other materials for the central layer 104 and/or the outer layers 102, 106 may be selected. The outer layers 102, 106 are configured to undergo no elastic deformation in response to the force.

The deferral of the first outer layer 102 relative to the second outer layer 106 may thereby be limited to 2 mm. Such deferral of maximum 2 mm may translate to a maximum movement of parts of the surgical imaging device of about 20 mm in the direction of the force. Such parts may be an arm 502 and/or an image acquisition apparatus carrier 504, e.g., a camera, optics carrier, or the like, of the surgical imaging device 500. Hence, in a scenario where the arm and/or image acquisition apparatus carrier 504 of the surgical imaging device 500 are hit (accidentally) by a user and undergo a typical displacement of 2 to 3 mm, the resulting displacement of the damper 100 would range between 0.2 to 0.3 mm. However, it is understood that the deferral of the damper 100 and the related displacement of parts of the surgical imaging device 500 may not be limited to a ratio of 1:10, but other ratios may be included by this disclosure in further embodiments.

Furthermore, the central layer 104 is configured to undergo deshearing following shearing deformation to return from a deformed state, which equals a sheared state of the damper 100 (see **Fig. 3** and **Fig. 4**), to an undeformed state, which equals an unsheared state of the damper 100 (see **Fig. 1** and **Fig. 2**). In an unsheared or undeformed state, the deferral of the outer layers 102, 106 may or may almost be 0 mm. The central layer 104 can repeatedly undergo shearing and deshearing in response to reoccurring pushing or pulling forces applied to the ends 110, 112. Deshearing may occur spontaneously after shearing of the central layer 104 because of the material properties of the central layer 104. Hence, use of an elastic material, such as polyurethane or materials of similar properties, may be preferred for the central layer 104. By returning to its desheared, undeformed state, the damper 100 may act on the stand 506 and/or the base 508 of the surgical imaging device 500 such that the surgical imaging device 500 may return to a steady state after the damper-deforming impact has occurred. This return to the steady state of the surgical imaging device 500 may only take one to a few seconds after the impact and with few or no extra cycles of back-and-forth movement of the respective parts 502, 504, the stand 506 and/or the base 508 of the surgical imaging device 500. Thereby, the surgical imaging device 500 becomes usable again instantly or quickly after an impact has occurred.

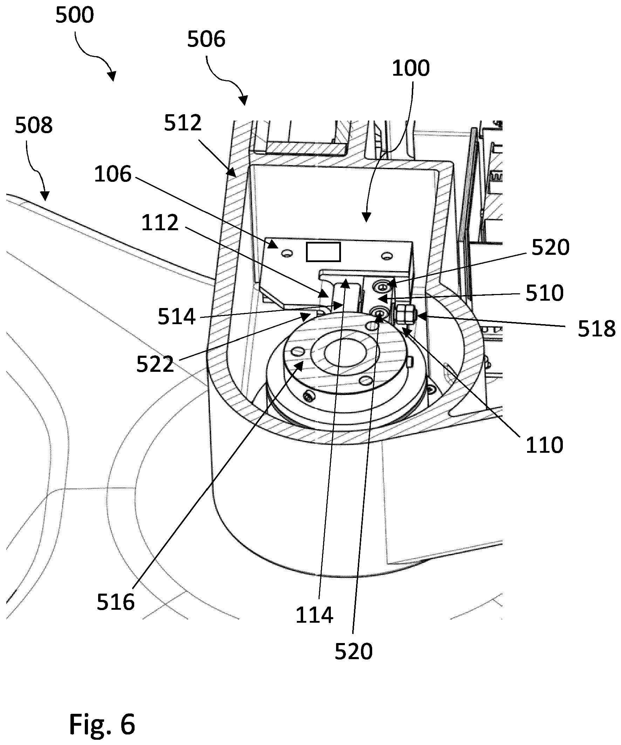

**Fig. 6** shows a perspective view of a damper 100 mounted within a surgical imaging device 500. The surgical imaging device 500 may comprise a fixation element 510 for fixing the first end 110 of the damper 100 to a housing 512 of a stand 506 of the surgical imaging device 500. The surgical imaging device 500 may further comprise a further fixation element 514 for fixing the second end 112 of the damper 100 to a connector part 516 of a base 508 of the surgical imaging device 500, with the connector part 516 configured to connect the base 508 with the stand 506. And the surgical imaging device 500 may comprise an axis 518 configured to movably connect the first end 110 and the second end 112. Thereby, both ends 110, 112 are extending from the same side 114 of the base piece 108 of the damper 100. The second end 112 is configured to be movably connected to the first end 110. The movement of the ends 110, 112 may occur along the axis 518 in the direction of the force. The fixation elements 510, 514 may be attached to the ends 110, 112 by mechanical means, such as screws 520 or a bolted connection 522, which may be gearing into respective openings 116 of the ends 110, 112 (see also **Fig. 1** and **Fig. 3**). By that the damper 100 may be mounted easily inside the surgical imaging device 500.

The surgical imaging device 500 may further comprise an image acquisition apparatus carrier 504 (see Fig. 5), such as a camera and/or an optics carrier, and an arm 502 (see Fig. 5). The damper 100 is thereby configured to undergo elastic deformation coaxially to a force exerted on the image acquisition apparatus carrier 504 and/or the arm 502. The force may be caused by a mechanical impact, such as a user (accidentally) hitting the image acquisition apparatus carrier 504 and/or the arm 502. The force is then transmitted through the surgical imaging device 500 as the image acquisition apparatus carrier 504, the arm 502, the stand 506, and the base 508 are mechanically linked. Therefore, an impact on the image acquisition apparatus carrier 504 and/or the arm 502 is eventually transmitted to the damper 100, which is connected to the stand 506 and the base 508 with its ends 110, 112.

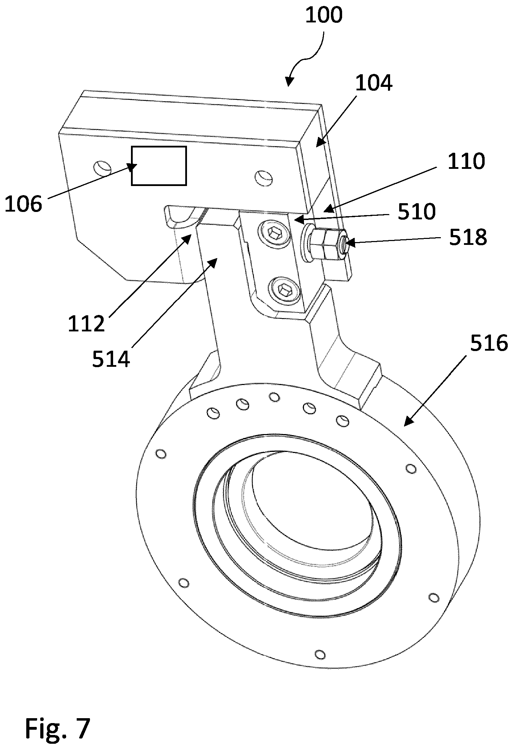

**Fig. 7** shows another perspective view of the damper 100 in the context of the surgical imaging device 500. The fixation element 510 is positioned at the first end 110 and the further fixation element 514 at the second end 112 with the axis 518 connecting both ends 110, 112. This is allowing for movement of the ends 110, 112 along the axis 518 in the direction of the force.

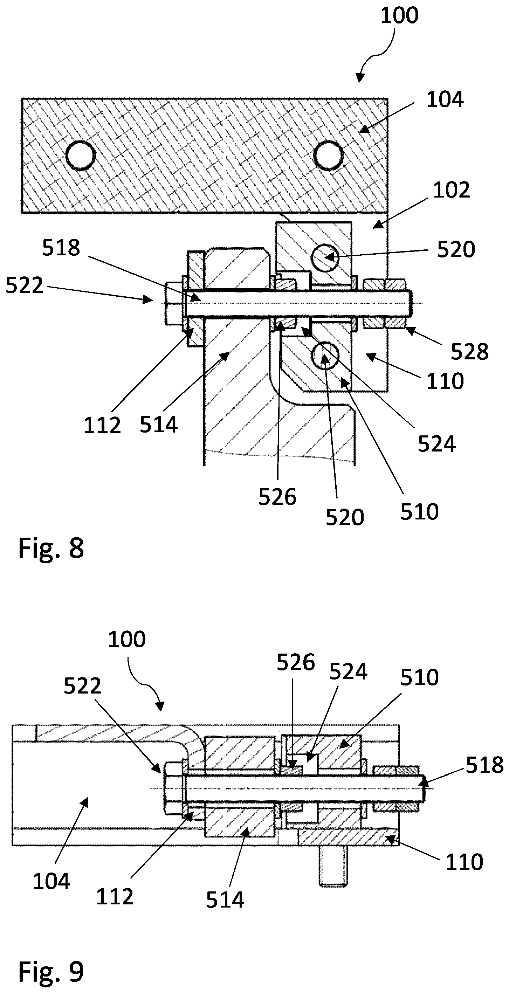

**Fig. 8** and **Fig. 9** show schematic sectional views of a damper 100 with **Fig. 8** showing a top section view and **Fig. 9** showing a side section view. The section plane is located at the center of the axis 518 (indicated by dashed lines in **Fig. 8** and **Fig. 9**). In the depicted embodiment, the first end 110 is configured to be fixed at the stand 506 by the fixation element 510. The second end 112 is configured to be fixed at the base 508 by the further fixation element 514. The second end 112 is further configured to be mounted on the axis 518, with the axis 518 being configured to engage with the fixation element 510 in such way that during deformation the first end 110 is displaced along the axis 518 thereby deferring the first outer layer 102 in direction of the force. The fixation elements 510 is attached to the end 110 by two screws 520. The further fixation element 514 is attached to the second end 112 by a bolted connection 522 along with the axis 518. In further embodiments the same or different fixation means may be utilized to attach the fixation elements 510, 514 to the ends 110, 112, which are known to a person skilled in the art. The fixation element 510 may comprise a cavity 524. The cavity 524 may allow for an at least partial take up of the further fixation element 514 and/or parts 526 of the bolted connection 522 when the first end 110 is displaced along the axis 518 towards the second end 112. By this a compact arrangement of the damper 100 inside the surgical imaging device 500 is achieved. The axis 518 may further comprise a mechanical stop 528. The mechanical stop 528 is positioned opposite of the second end 112 and the fixation elements 510, 514. The mechanical stop 528 thereby limits the displacement of the first end 110 along the axis 518. In the depicted embodiment the displacement may be limited such that the deferral of the outer layers 102, 106 is limited to a maximum of 2 mm. Other limits of displacement and therefore other positions of the mechanical stop 528 on the axis 518 may be used in further embodiments. The use of the mechanical stop 528 may prevent over-shearing of the central layer 104 and by that damaging of the damper 100.

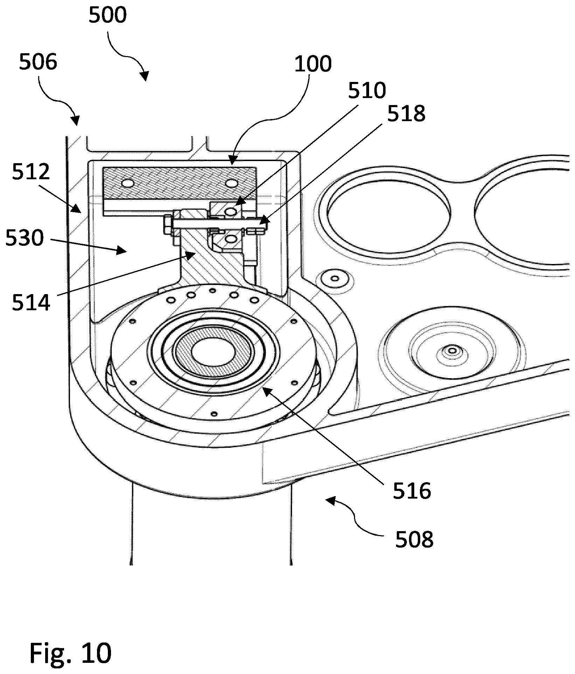

**Fig. 10** shows a sectional view of a surgical imaging device 500. The section plane is at the center of the axis 518 (similar to **Fig. 8**)**.** In the embodiment, the fixation element 510 is fixing the first end 110 of the damper 100 to the housing 512 of a stand 506. The housing 512 thereby comprises a compartment 530 for taking up the damper 100. The further fixation element 514 is fixing the second end 112 of the damper 100 to the connector part 516 of the base 508. The connector part 516 is configured to connect the base 508 with the stand 506. A movement of the stand 506 and/or the base 508 exerts a pushing or pulling force on at least one of the ends 110, 112 of the damper 100, hence, leading to its deformation.

As used herein the term "and/or" includes any and all combinations of one or more of the associated listed items and may be abbreviated as "/".

### List of Reference Signs

- 100: Damper
- 102: Outer layer
- 104: Central layer
- 106: Outer layer
- 108: Base piece
- 110: First end
- 112: Second end
- 114: Side
- 116: Opening

- 500: Surgical imaging device
- 502: Arm
- 504: Image acquisition apparatus carrier
- 506: Stand
- 508: Base
- 510: Fixation element
- 512: Housing
- 514: Fixation element
- 516: Connector part
- 518: Axis
- 520: Screw
- 522: Bolted connection
- 524: Cavity
- 526: Part
- 528: Mechanical stop
- 530: Compartment

## Claims

1. Damper for a surgical imaging device (500), the damper (100) comprising a stack of layers (102, 104, 106) comprising: a first outer layer (102), a central layer (104), and a second outer layer (106); wherein the layers are linked to form a base piece (108) of the damper (100); wherein the first outer layer (102) further forms a first end (110) of the damper (100) configured to be connected to a stand (506) of the surgical imaging device (500) and the second outer layer (106) further forms a second end (112) of the damper (100) configured to be connected to a base (508) of the surgical imaging device (500); wherein the damper (100) is configured to undergo elastic deformation in response to a pushing or pulling force exhibited on one of the ends (110, 112) such that the central layer (104), but not the outer layers (102, 106), undergo elastic deformation; wherein application of the force to the first end (110) causes a deferral of the first outer layer (102) relative to the second outer layer (106), thereby inducing shearing deformation of the central layer (104),
**characterized in that**
the ends (110, 112) are extending from the same side (114) of the base piece (108) of the damper (100),
wherein the second end (112) is configured to be movably connected to the first end (110), wherein the first end (110) is configured to be fixed at the stand (506) by a fixation element (510),
wherein the second end (112) is configured to be fixed at the base (508) by at least one further fixation element (514), and
wherein the second end (112) is further configured to be mounted on an axis (518), with the axis (518) being configured to engage with the fixation element (510) in such way that during deformation the first end (110) is displaced along the axis (518) thereby deferring the first outer layer (102) in direction of the force.

2. Damper according to claim 1, wherein the deferral is limited to 2 mm.

3. Damper according to one of the preceding claims, wherein the central layer (104) undergoes shearing deformation coaxially to a displacement of the first end (110).

4. Damper according to one of the preceding claims, wherein the outer layers (102, 106) are made of sheet metal, wherein the central layer (104) is made of an elastomer, preferably polyurethane.

5. Damper according to one of the preceding claims, the central layer (104) is configured to undergo deshearing following shearing deformation to return from a deformed state to an undeformed state.

6. Surgical imaging device comprising a stand (506), a base (508), a damper (100) according to one of the preceding claims, a fixation element (510) for fixing a first end (110) of the damper (100) to a housing (512) of the stand (506), a further fixation element (514) for fixing a second end (112) of the damper (100) to a connector part (516) of the base (508), with the connector part (516) configured to connect the base (508) with the stand (506), and an axis (518) configured to movably connect the first end (110) and the second end (112).

7. Surgical imaging device according to claim 6, the surgical imaging device (500) further comprising an image acquisition apparatus carrier (504) and an arm (502), wherein the damper (100) is configured to undergo elastic deformation coaxially to a force exerted on the image acquisition apparatus carrier (504) and/or the arm (502).

## Patentansprüche

1. Dämpfer für eine chirurgische Bildgebungsvorrichtung (500), wobei der Dämpfer (100) einen Schichtstapel (102, 104, 106) umfasst, umfassend: eine erste Außenschicht (102), eine Mittelschicht (104) und eine zweite Außenschicht (106); wobei die Schichten verbunden sind, um ein Basisteil (108) des Dämpfers (100) zu bilden; wobei die erste Außenschicht (102) ferner ein erstes Ende (110) des Dämpfers (100) bildet, das ausgebildet ist, mit einem Stativ (506) der chirurgischen Bildgebungsvorrichtung (500) verbunden zu werden, und die zweite Außenschicht (106) ferner ein zweites Ende (112) des Dämpfers (100) bildet, das ausgebildet ist, mit einem Sockel (508) der chirurgischen Bildgebungsvorrichtung (500) verbunden zu werden; wobei der Dämpfer (100) ausgebildet ist, als Reaktion auf eine an einem der Enden (110, 112) ausgeübte Druck- oder Zugkraft eine elastische Verformung zu durchlaufen, sodass die Mittelschicht (104), jedoch nicht die Außenschichten (102, 106), eine elastische Verformung durchläuft; wobei die Anwendung der Kraft auf das erste Ende (110) einen Versatz der ersten Außenschicht (102) relativ zur zweiten Außenschicht (106) veranlasst, wodurch eine Scherverformung der Mittelschicht (104) induziert wird,
**dadurch gekennzeichnet, dass**
die Enden (110, 112) sich von derselben Seite (114) des Basisteils (108) des Dämpfers (100) erstrecken,
wobei das zweite Ende (112) ausgebildet ist, beweglich mit dem ersten Ende (110) verbunden zu werden,
wobei das erste Ende (110) ausgebildet ist, am Stativ (506) durch ein Befestigungselement (510) befestigt zu werden,
wobei das zweite Ende (112) ausgebildet ist, am Sockel (508) durch zumindest ein weiteres Befestigungselement (514) befestigt zu werden, und
wobei das zweite Ende (112) ferner ausgebildet ist, auf einer Achse (518) montiert zu werden, wobei die Achse (518) ausgebildet ist, mit dem Befestigungselement (510) in einer solchen Weise in Eingriff zu gelangen, dass während der Verformung das erste Ende (110) entlang der Achse (518) verschoben wird, wodurch die erste Außenschicht (102) in Richtung der Kraft versetzt wird.

2. Dämpfer nach Anspruch 1, wobei der Versatz auf 2 mm begrenzt ist.

3. Dämpfer nach einem der vorstehenden Ansprüche, wobei die Mittelschicht (104) eine Scherverformung koaxial zu einer Verschiebung des ersten Endes (110) durchläuft.

4. Dämpfer nach einem der vorstehenden Ansprüche, wobei die Außenschichten (102, 106) aus Blech hergestellt sind, wobei die Mittelschicht (104) aus einem Elastomer hergestellt ist, bevorzugt Polyurethan.

5. Dämpfer nach einem der vorstehenden Ansprüche, wobei die Mittelschicht (104) ausgebildet ist, nach einer Scherverformung ein Entscheren zu durchlaufen, um von einem verformten Zustand in einen unverformten Zustand zurückzukehren.

6. Chirurgische Bildgebungsvorrichtung umfassend ein Stativ (506), einen Sockel (508), einen Dämpfer (100) nach einem der vorstehenden Ansprüche, ein Befestigungselement (510) zum Befestigen eines ersten Endes (110) des Dämpfers (100) an einem Gehäuse (512) des Stativs (506), ein weiteres Befestigungselement (514) zum Befestigen eines zweiten Endes (112) des Dämpfers (100) an einem Verbindungsteil (516) des Sockels (508), wobei das Verbindungsteil (516) ausgebildet ist, den Sockel (508) mit dem Stativ (506) zu verbinden, und eine Achse (518), die ausgebildet ist, das erste Ende (110) und das zweite Ende (112) beweglich zu verbinden.

7. Chirurgische Bildgebungsvorrichtung nach Anspruch 6, die chirurgische Bildgebungsvorrichtung (500) weiter einen Bildaufnahmeeinrichtungsträger (504) und einen Arm (502) umfasst, wobei der Dämpfer (100) ausgebildet ist, eine elastische Verformung koaxial zu einer auf den Bildaufnahmeeinrichtungsträger (504) und/oder den Arm (502) ausgeübten Kraft zu durchlaufen.

## Revendications

1. Amortisseur pour dispositif d'imagerie chirurgicale (500), l'amortisseur (100) comprenant un empilement de couches (102, 104, 106) comprenant : une première couche externe (102), une couche centrale (104) et une deuxième couche externe (106) ; dans lequel les couches sont liées pour former une pièce de base (108) de l'amortisseur (100) ; dans lequel la première couche externe (102) forme en outre une première extrémité (110) de l'amortisseur (100) configurée pour être reliée à un support (506) du dispositif d'imagerie chirurgicale (500) et la deuxième couche externe (106) forme en outre une deuxième extrémité (112) de l'amortisseur (100) configurée pour être reliée à un socle (508) du dispositif d'imagerie chirurgicale (500) ; dans lequel l'amortisseur (100) est configuré pour subir une déformation élastique en réponse à une force de poussée ou de traction exercée sur l'une des extrémités (110, 112) de telle sorte que la couche centrale (104), mais non les couches externes (102, 106), subit une déformation élastique ; dans lequel l'application de la force à la première extrémité (110) provoque un déplacement de la première couche externe (102) par rapport à la deuxième couche externe (106), induisant ainsi une déformation par cisaillement de la couche centrale (104),
**caractérisé en ce que**
les extrémités (110, 112) s'étendent depuis le même côté (114) de la pièce de base (108) de l'amortisseur (100),
dans lequel la deuxième extrémité (112) est configurée pour être reliée de manière mobile à la première extrémité (110),
dans lequel la première extrémité (110) est configurée pour être fixée au support (506) par un élément de fixation (510),
dans lequel la deuxième extrémité (112) est configurée pour être fixée au socle (508) par au moins un élément de fixation supplémentaire (514), et
dans lequel la deuxième extrémité (112) est en outre configurée pour être montée sur un axe (518), l'axe (518) étant configuré pour s'engager avec l'élément de fixation (510) de telle sorte que, pendant la déformation, la première extrémité (110) est déplacée le long de l'axe (518), déplaçant ainsi la première couche externe (102) dans la direction de la force.

2. Amortisseur selon la revendication 1, dans lequel le déplacement est limité à 2 mm.

3. Amortisseur selon l'une quelconque des revendications précédentes, dans lequel la couche centrale (104) subit une déformation par cisaillement coaxialement à un déplacement de la première extrémité (110).

4. Amortisseur selon l'une quelconque des revendications précédentes, dans lequel les couches externes (102, 106) sont en tôle métallique, dans lequel la couche centrale (104) est faite d'un élastomère, de préférence en polyuréthane.

5. Amortisseur selon l'une quelconque des revendications précédentes, la couche centrale (104) est configurée pour subir un dé-cisaillement à la suite d'une déformation par cisaillement afin de revenir d'un état déformé à un état non déformé.

6. Dispositif d'imagerie chirurgicale comprenant un support (506), un socle (508), un amortisseur (100) selon l'une quelconque des revendications précédentes, un élément de fixation (510) pour fixer une première extrémité (110) de l'amortisseur (100) à un boîtier (512) du support (506), un élément de fixation supplémentaire (514) pour fixer une deuxième extrémité (112) de l'amortisseur (100) à une pièce de liaison (516) du socle (508), la pièce de liaison (516) étant configurée pour relier le socle (508) au support (506), et un axe (518) configuré pour relier de manière mobile la première extrémité (110) et la deuxième extrémité (112).

7. Dispositif d'imagerie chirurgicale selon la revendication 6, le dispositif d'imagerie chirurgicale (500) comprenant en outre un support d'appareil d'acquisition d'images (504) et un bras (502), dans lequel l'amortisseur (100) est configuré pour subir une déformation élastique coaxialement à une force exercée sur le support d'appareil d'acquisition d'images (504) et/ou le bras (502).
